# FASCICULE DE BREVET EUROPEEN

(11) **EP 3 522 955 B1**
(45) Date de publication et mention de la délivrance du brevet: **12.08.2020**
(21) Numéro de dépôt: 17793988.1
(22) Date de dépôt: 02.10.2017
(51) Int. Cl.: A61M 5/31, A61M 5/32

(54) **DISPOSITIF D'INJECTION MANUELLE.**
MANUELLE INJEKTIONSVORRICHTUNG
MANUAL INJECTION DEVICE

(30) Priorité: 04.10.2016 FR 1659549
(43) Date de publication de la demande: 14.08.2019
(73) Titulaire: Aptar France SAS, 27110 Le Neubourg (FR)
(72) Inventeur: JAOUEN, Quentin, 76220 Gournay en Bray (FR); SAUSSAYE, Anthony, 27400 Louviers (FR)
(74) Mandataire: CAPRI
(86) Numéro de dépôt international: PCT/FR2017/052698
(87) Numéro de publication internationale: WO 2018/065714

(56) Documents cités:
- EP-A1- 2 572 741
- WO-A1-2006/129196
- WO-A1-2012/045350
- WO-A1-2014/150201
- WO-A2-2014/053378
- FR-A1- 2 884 722
- FR-A1- 3 019 748
- US-A- 5 320 609

## Description

La présente invention concerne un dispositif d'injection manuelle.

Les dispositifs d'injection manuelle ont principalement pour but de réaliser la pénétration de l'aiguille dans le corps du patient ainsi que la protection de l'aiguille de la seringue avant, pendant et après l'injection. Par contre, l'injection dans le corps du patient du produit contenu dans la seringue est réalisée manuellement par l'utilisateur. Les dispositifs d'injection manuelle sont des dispositifs relativement complexes qui doivent répondre à un certain nombre d'exigences de contraintes pour être fiables. La robustesse du dispositif, sa maniabilité, et sa facilité d'utilisation pour l'utilisateur sont également des éléments importants. Par ailleurs, la plupart de ces dispositifs d'injection manuelle étant à usage unique, le coût de fabrication et d'assemblage est également un facteur dont il faut tenir compte.

Il existe de nombreux dispositifs d'injection manuelle sur le marché, qui présentent toutefois tous un certain nombre d'inconvénients.

Ainsi, il ne faut pas que l'utilisation du dispositif d'injection manuelle soit trop difficile, ce qui empêcherait des personnes faibles de l'utiliser. Il faut aussi éviter que l'injection ne commence avant que l'aiguille ait pénétré dans le corps du patient. De plus, afin d'éviter tout risque de blessures avant et après l'utilisation du dispositif, le dispositif d'injection manuelle doit comprendre un dispositif de sécurité aiguille qui évite que l'aiguille ne soit apparente avant et après l'utilisation du dispositif. Ce dispositif de sécurité doit évidemment également être fiable et ne pas se libérer trop facilement. Il doit aussi être fonctionnel même si l'utilisateur actionne mal le dispositif d'injection manuelle, par exemple s'il le retire trop tôt de son corps, avant la fin de l'injection.

Les documents WO2014150201, WO2011047298, WO2012045350, WO2006129196, WO2006111862, FR2884722, US5320609, EP2572741 et FR3019748 décrivent des dispositifs de l'état de la technique.

La présente invention a pour but de fournir un dispositif d'injection manuelle qui ne reproduit pas les inconvénients susmentionnés, et qui permet de répondre aux différentes exigences et contraintes importantes pour une utilisation sûre et fiable du dispositif d'injection manuelle.

La présente invention a notamment but de fournir un dispositif d'injection manuelle qui évite le risque que l'injection du produit ne débute avant que l'aiguille ait complètement pénétrée le site d'injection.

La présente invention a aussi pour but de fournir un dispositif d'injection manuelle qui soit fiable d'utilisation, qui soit sûr et qui empêche tout risque de blessure, et qui soit simple et peu coûteux à fabriquer et à assembler.

La présente invention a donc pour objet un dispositif d'injection manuelle comportant :
- un corps inférieur recevant un réservoir, ledit réservoir étant monté axialement déplaçable dans ledit corps inférieur et contenant du produit fluide à injecter, ledit réservoir comportant un piston et une aiguille,
- un corps supérieur monté axialement déplaçable par rapport audit corps inférieur lors de l'actionnement, ledit corps supérieur comportant une tige de piston coopérant avec ledit piston lors de l'injection pour le déplacer dans le réservoir,
- un manchon monté déplaçable par rapport audit corps inférieur entre une position de repos, dans laquelle ledit manchon est disposé à l'intérieur dudit corps inférieur, et une position projetée, dans laquelle ledit manchon est axialement déplacé hors dudit corps inférieur, ledit manchon étant bloqué dans sa position de repos avant actionnement du dispositif d'injection manuelle et étant sollicité automatiquement dans sa position projetée après injection,
- ladite tige de piston coopérant avec ledit réservoir, ou avec tout élément solidaire dudit réservoir, pour former une serrure d'injection, la force nécessaire pour déplacer axialement ledit réservoir dans ledit corps inférieur étant inférieure à la force nécessaire pour déclencher ladite serrure d'injection, de sorte que ledit piquage est réalisé avant ladite injection,
dans lequel, avant actionnement, ladite tige de piston est reliée à une bague fixée, notamment sertie, sur une collerette radiale dudit réservoir.

Avantageusement, ladite tige de piston, avant actionnement, est reliée par des ponts sécables à ladite bague.

En variante, ladite bague comporte une projection radialement interne coopérant, avant actionnement, avec une gorge de ladite tige de piston.

Avantageusement, ledit manchon comporte une extrémité élastiquement déformable radialement vers l'intérieur, ladite extrémité reposant en position de repos sur un épaulement radial du corps inférieur, ledit corps supérieur coopérant en fin d'injection via une came avec ladite extrémité dudit manchon, pour déformer ladite extrémité radialement vers l'intérieur.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
la figure 1 est une vue schématique en section transversale d'un dispositif d'injection manuelle selon un mode de réalisation avantageux, en position de repos avant piquage,
la figure 2 est une vue similaire à celle de la figure 1, selon un autre angle de vue,
la figure 3 est une vue similaire à celle de la figure 2, en fin de piquage, avant déclenchement de la serrure d'injection,
la figure 4 est une vue similaire à celle de la figure 3, après déclenchement de la serrure d'injection,
la figure 5 est une vue similaire à celle de la figure 4, en fin d'injection,
la figure 6 est une vue similaire à celle de la figure 5, après déclenchement du dispositif de sécurité post-injection,
la figure 7 est une vue de détail d'une première variante de réalisation de la serrure d'injection,
la figure 8 est une vue de détail d'une seconde variante de réalisation de la serrure d'injection, et
les figures 9 et 10 sont des vues schématiques de détail du déclenchement du dispositif de sécurité post-injection.

Le dispositif d'injection manuelle représenté sur les figures comporte un corps inférieur 1 et un corps supérieur 2, axialement déplaçable par rapport audit corps inférieur 1 lors de l'actionnement. Il est à noter que les corps inférieur 1 et supérieur 2 peuvent être réalisés de manière monobloc ou en plusieurs parties assemblées. De manière connue, ce corps inférieur 1 contient un réservoir S contenant le produit à injecter, une aiguille A fixée audit réservoir S à travers laquelle le produit est distribué et un piston P adapté à se déplacer dans ledit réservoir S pour réaliser cette injection. Le corps supérieur 2 comporte une tige de piston TP coopérant avec ledit piston P lors de l'injection pour le déplacer dans le réservoir S. L'aiguille A peut être protégée avant utilisation par un capuchon de protection. Le réservoir S peut typiquement être une seringue pré-remplie classique, pourvue d'une collerette radiale 9. Ledit réservoir S est mobile axialement dans ledit corps inférieur 1 pour réaliser le piquage, puis est fixe par rapport audit corps inférieur 1 lors de l'injection. Ladite tige de piston TP est fixe par rapport audit corps supérieur 2. Ainsi, lorsque le corps supérieur 2 coulisse axialement par rapport au corps inférieur 1, la tige de piston TP coulisse axialement par rapport au réservoir S.

Avant actionnement, un manchon 10 de sécurité post-injection est disposé à l'intérieur du corps inférieur. Après l'injection, ledit manchon 10 est déplacé axialement hors du corps inférieur 1 dans une position projetée, dans laquelle il est disposé autour de l'aiguille A, pour éviter tout risque de blessure avec ladite aiguille A, formant ainsi un dispositif de sécurité post-injection. Le manchon 10 est avantageusement sollicité vers sa position projetée par un ressort, qui peut être de type quelconque. Avant actionnement, ledit manchon 10 est bloqué et empêché de se déplacer vers sa position projetée par des moyens de blocage qui sont libérés en fin d'injection.

La tige de piston TP définit avec le réservoir S (ou avec tout élément solidaire dudit réservoir S, tel que par exemple un support de réservoir ou une bague encliquetée sur ledit réservoir) une serrure d'actionnement. Cette serrure comprend une serrure de piquage et une serrure d'injection.

Selon l'invention, la serrure de piquage est actionnée avant la serrure d'injection, c'est à dire que la force axiale nécessaire pour déplacer axialement le réservoir S dans le corps inférieur 1 est inférieure à la force axiale nécessaire pour déclencher la serrure d'injection. Ainsi, lorsque l'utilisateur appuie le dispositif contre le site d'injection et appuie axialement sur le corps supérieur 2 pour le faire coulisser axialement par rapport au corps inférieur 1, c'est d'abord le réservoir S qui se déplace axialement à l'intérieur du corps inférieur 1 pour réaliser le piquage. La tige de piston TP ne se déplace axialement à l'intérieur du réservoir S qu'après que le piquage est terminé.

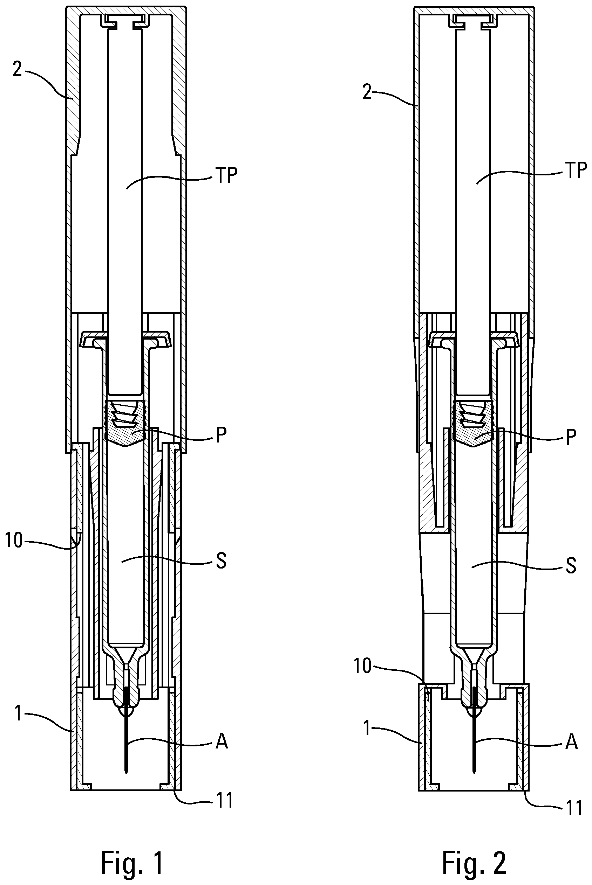

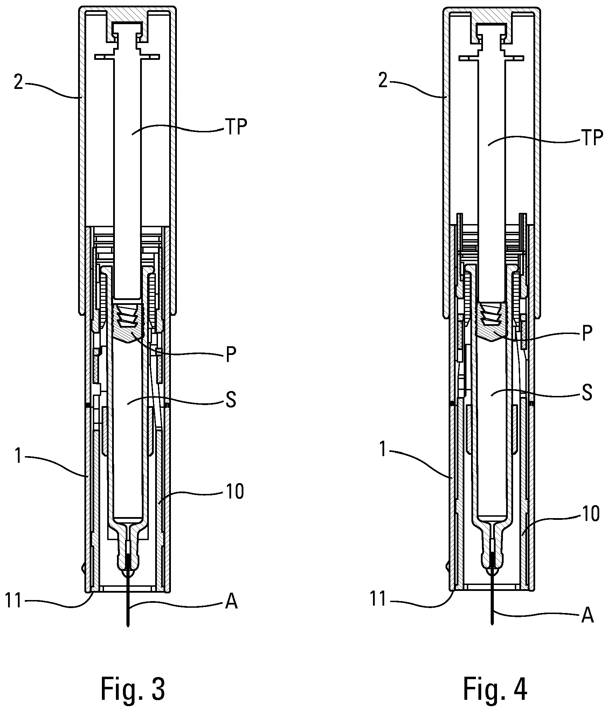

Les figures 1 à 6 illustrent un mode de réalisation avantageux et montrent différentes positions dans la séquence d'actionnement du dispositif d'injection manuelle.

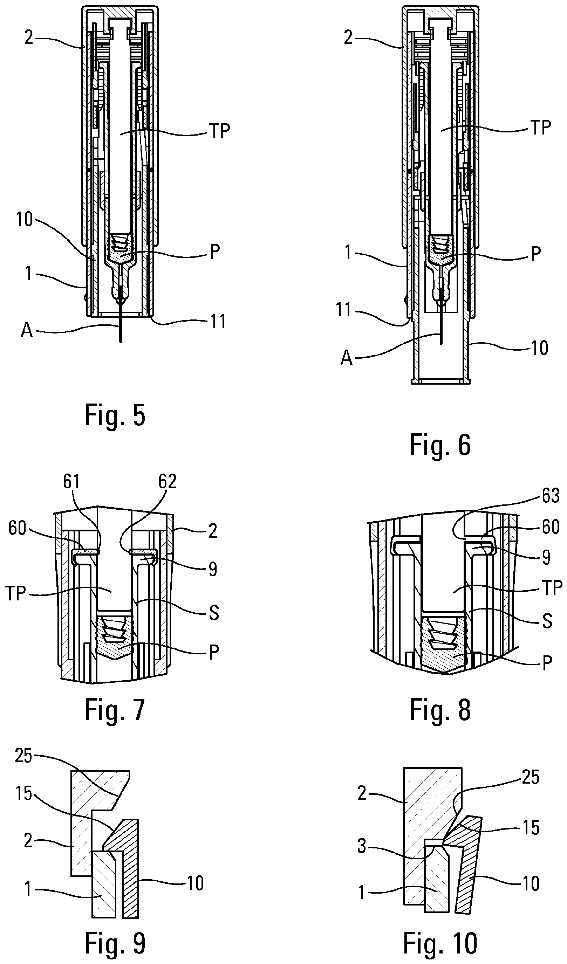

En référence aux figures 1 à 6, l'utilisateur appuie l'extrémité inférieure 11 du corps inférieur 1 autour du site d'injection, et exerce une pression axiale sur le corps supérieur 2. La résistance au déclenchement de la serrure d'injection, qui sera décrite ultérieurement, étant supérieure à la résistance au déplacement axial du réservoir S dans ledit corps inférieur 1, c'est d'abord le réservoir S qui va coulisser axialement vers l'intérieur du corps inférieur 1. Ceci va permettre à l'aiguille A de pénétrer dans le site d'injection sous l'effet de ladite pression axiale de l'utilisateur. Ce n'est que lorsque le réservoir S atteint sa position piquée, en butée axiale dans le corps inférieur 1, empêchant ainsi tout déplacement axial supplémentaire dudit réservoir S, que la force d'actionnement ou pression axiale de l'utilisateur servira à déclencher la serrure d'injection. Après déclenchement de ladite serrure d'injection, le corps supérieur 2 et donc la tige de piston TP vont se déplacer axialement par rapport au réservoir S, pour déplacer le piston P dans le réservoir S et ainsi injecter le produit contenu dans le réservoir, à travers l'aiguille A, dans le site d'injection. En fin d'injection, lorsque l'utilisateur retire le dispositif d'injection manuelle du site d'injection, le manchon 10 est libéré des moyens de blocage et se déplace automatiquement, par exemple sous l'effet du ressort de rappel 5, vers sa position projetée, dans laquelle il se verrouille, pour éviter tout risque de blessure avec l'aiguille A, comme visible sur la figure 6.

La figure 7 illustre une première variante de réalisation de la serrure d'injection. Dans cette variante, la serrure d'injection est formée entre la tige de piston TP et une bague 60 fixée, notamment sertie, directement sur la collerette radiale 9 de la seringue S. Ladite bague 60 comporte une projection radialement interne 61 fixée à ladite tige de piston TP par des ponts sécables 63. La résistance à la rupture de ces ponts sécables 63 est supérieure à la force de déclenchement de la serrure de piquage, c'est-à-dire au déplacement axial du réservoir S dans le corps inférieur 1, de sorte que le piquage a lieu avant l'injection.

La figure 8 illustre une autre variante dans laquelle ladite projection radialement interne 61 de bague de sertissage 60 coopère avant actionnement avec une gorge 62 de la tige de piston TP. La force nécessaire pour désengager ladite projection 61 de ladite gorge 62 est supérieure à la force de déclenchement de la serrure de piquage, c'est-à-dire au déplacement axial du réservoir S dans le corps inférieur 1, de sorte que le piquage a lieu avant l'injection.

D'autres variantes de réalisation de la serrure de piquage et/ou de la serrure d'injection sont aussi envisageables.

Les figures 9 et 10 illustrent le déclenchement du dispositif de sécurité post-injection, avec le manchon 10 qui se déplace hors du corps inférieur 1 vers sa position projetée. Le manchon 10 comporte une extrémité 15 élastiquement déformable radialement vers l'intérieur. En position de repos, représentée sur la figure 9, cette extrémité 15 repose sur un épaulement radial 3 du corps inférieur 1. Le manchon 10 est donc bloqué contre tout déplacement axial vers sa position projetée par rapport audit corps inférieur 1. En fin d'injection, le corps supérieur 2 vient coopérer via une came 25 avec ladite extrémité 15 du manchon, pour la déformer radialement vers l'intérieur, comme visible sur la figure 10. Le manchon 10 n'est alors plus bloqué, et il peut se déplacer vers sa position projetée, par exemple sous l'effet d'un ressort approprié.

Bien que la présente invention ait été décrite en référence à des modes et variantes de réalisation avantageux, il est entendu que l'homme du métier peut y apporter diverses modifications sans sortir du cadre de la présente invention tel que défini par les revendications annexées.

## Revendications

1. Dispositif d'injection manuelle comportant :
- un corps inférieur (1) recevant un réservoir (S), ledit réservoir étant monté axialement déplaçable dans ledit corps inférieur (1) et contenant du produit fluide à injecter, ledit réservoir (S) comportant un piston (P) et une aiguille (A),
- un corps supérieur (2) monté axialement déplaçable par rapport audit corps inférieur (1) lors de l'actionnement, ledit corps supérieur (2) comportant une tige de piston (TP) coopérant avec ledit piston (P) lors de l'injection pour le déplacer dans le réservoir (S),
- un manchon (10) monté déplaçable par rapport audit corps inférieur (1) entre une position de repos, dans laquelle ledit manchon (10) est disposé à l'intérieur dudit corps inférieur (1), et une position projetée, dans laquelle ledit manchon (10) est axialement déplacé hors dudit corps inférieur (1), ledit manchon (10) étant bloqué dans sa position de repos avant actionnement du dispositif d'injection manuelle et étant sollicité automatiquement dans sa position projetée après injection,
**caractérisé en ce qu'**avant actionnement, ladite tige de piston (TP) est reliée à une bague (60) fixée, notamment sertie, sur une collerette radiale (9) dudit réservoir (S) pour former une serrure d'injection, la force nécessaire pour déplacer axialement ledit réservoir (S) dans ledit corps inférieur (1) étant inférieure à la force nécessaire pour déclencher ladite serrure d'injection, de sorte que ledit piquage est réalisé avant ladite injection.

2. Dispositif d'injection manuelle selon la revendication 1, dans lequel ladite tige de piston (TP), avant actionnement, est reliée par des ponts sécables (63) à ladite bague (60).

3. Dispositif d'injection manuelle selon la revendication 1, dans lequel ladite bague (60) comporte une projection radialement interne (61) coopérant, avant actionnement, avec une gorge (62) de ladite tige de piston (TP).

4. Dispositif d'injection manuelle selon l'une quelconque des revendications précédentes, dans lequel ledit manchon (10) comporte une extrémité (15) élastiquement déformable radialement vers l'intérieur, ladite extrémité (15) reposant en position de repos sur un épaulement radial (3) du corps inférieur (1), ledit corps supérieur (2) coopérant en fin d'injection via une came (25) avec ladite extrémité (15) dudit manchon (10), pour déformer ladite extrémité (15) radialement vers l'intérieur.

## Patentansprüche

1. Manuelle Injektionsvorrichtung, umfassend
- einen unteren Körper (1), in dem ein Vorratsbehälter (S) aufgenommen ist, wobei der Vorratsbehälter axial verschiebbar in dem unteren Körper (1) angebracht ist und ein zu injizierendes Flüssigprodukt enthält, wobei der Vorratsbehälter (S) einen Kolben (P) und eine Nadel (A) aufweist,
- einen oberen Körper (2), der bei Betätigung in Bezug auf den unteren Körper (1) axial verschiebbar ist, wobei der obere Körper (2) eine Kolbenstange (TP) aufweist, die beim Injektionsvorgang mit dem Kolben (P) zusammenwirkt, um diesen in dem Vorratsbehälter (S) zu verschieben,
- eine Hülse (10), die in Bezug auf den unteren Körper (1) zwischen einer Ruhestellung, in der die Hülse (10) im Inneren des unteren Körpers (1) angeordnet ist, und einer vorstehenden Stellung, in der die Hülse (10) axial aus dem unteren Körper (1) heraus verschoben ist, verschiebbar angebracht ist, wobei die Hülse (10) vor Betätigung der manuellen Injektionsvorrichtung in ihrer Ruhestellung arretiert ist und nach der Injektion automatisch in ihre vorstehende Stellung gedrückt wird,
**dadurch gekennzeichnet, dass** vor der Betätigung die Kolbenstange (TP) mit einem Ring (60) verbunden ist, der an einem radialen Kragen (9) des Vorratsbehälters (S) angebracht, insbesondere aufgepresst ist, um eine Injektionssperre zu bilden, wobei die zur axialen Verschiebung des Vorratsbehälters (S) im unteren Körper (1) erforderliche Kraft geringer ist als die zur Lösung der Injektionssperre erforderliche Kraft, sodass der Einstechvorgang vor der Injektion erfolgt.

2. Manuelle Injektionsvorrichtung nach Anspruch 1, bei der die Kolbenstange (TP) vor der Betätigung durch abtrennbare Stege (63) mit dem Ring (60) verbunden ist.

3. Manuelle Einspritzvorrichtung nach Anspruch 1, bei der der Ring (60) einen radial inneren Vorsprung (61) aufweist, der vorder Betätigung mit einer Nut (62) der Kolbenstange (TP) zusammenwirkt.

4. Manuelle Injektionsvorrichtung nach einem der voranstehenden Ansprüche, bei der die Hülse (10) ein Ende (15) aufweist, das radial nach innen elastisch verformbar ist, wobei das Ende (15) in der Ruhestellung an einer radialen Schulter (3) des unteren Körpers (1) anliegt, wobei der obere Körper (2) am Ende des Injektionsvorgangs über einen Nocken (25) mit dem Ende (15) der Hülse (10) zusammenwirkt, um das Ende (15) radial nach innen zu verformen.

## Claims

1. A manual injection device comprising:
- a lower body (1) that receives a reservoir (S), said reservoir being mounted to move axially in said lower body (1) and containing fluid to be injected, said reservoir (S) including a piston (P) and a needle (A),
- an upper body (2) that is mounted to move axially relative to said lower body (1) during actuation, said upper body (2) including a piston rod (TP) co-operating with said piston (P) during injection so as to move it in the reservoir (S),
- a sleeve (10) that is mounted to move relative to said lower body (1) between a rest position, in which said sleeve (10) is arranged inside said lower body (1), and a projecting position, in which said sleeve (10) is moved axially out of said lower body (1), said sleeve (10) being blocked in its rest position before the manual injection device is actuated, and being urged automatically into its projecting position after injection,
the manual injection device being **characterized in that**, before actuation, said piston rod (TP) is connected to a ring (60) fastened, in particular crimped, on a radial collar (9) of said reservoir (S) so as to form an injection lock, the force necessary for moving said reservoir (S) axially in said lower body (1) being less than the force necessary for triggering said injection lock, such that said pricking is performed before said injection.

2. A manual injection device according to claim 1, wherein said piston rod (TP), before actuation, is connected via breakable bridges (63) to said ring (60).

3. A manual injection device according to claim 1, wherein said ring (60) includes a radially-inner projection (61) co-operating, before actuation, with a groove (62) of said piston rod (TP).

4. A manual injection device according to any preceding claim, wherein said sleeve (10) includes an end (15) elastically deformable in a radially-inward direction, said end (15) in its rest position bearing against a radial shoulder (3) of the lower body (1), said upper body (2) co-operating at the end of injection, via a cam (25) with said end (15) of said sleeve (10), so as to deform said end (15) in a radially-inward direction.
